# EUROPÄISCHE PATENTSCHRIFT

(11) **EP 0 334 154 B1**
(45) Veröffentlichungstag und Bekanntmachung des Hinweises auf die Patenterteilung: **12.05.1993**
(21) Anmeldenummer: 89104407.5
(22) Anmeldetag: 13.03.1989
(51) Int. Cl.: C07C 67/08

(54) **Verfahren zur kontinuierlichen Veresterung von Fettsäuren**
Method for the continuous esterification of fatty acids
Procédé pour l'estérification continueuse d'acides gras

(30) Priorität: 21.03.1988 DE 3809417
(43) Veröffentlichungstag der Anmeldung: 27.09.1989
(73) Patentinhaber: Henkel Kommanditgesellschaft auf Aktien, 40191 Düsseldorf (DE)
(72) Erfinder: Bremus, Norbert, D-4018 Langenfeld (DE); Gutsche, Bernhard, Dr., D-4010 Hilden (DE); Jeromin, Lutz, Dr., D-4010 Hilden (DE); Peukert, Eberhard, D-4010 Hilden (DE); Schleper, Bernard, D-4000 Düsseldorf 1 (DE)

(56) Entgegenhaltungen:
- EP-A- 0 033 929
- EP-A- 0 082 301
- DE-B- 2 503 195
- CHEMIKER ZEITUNG - CHEMISCHE APPARATUR, 87. Jahrgang, Nr. 18, 1963, Seiten 661-666, Dr. Alfred Huthig Verlag Gmbh, Heidelberg, D ; H. STAGE et al.: "Fettsäureveresterung in Gegenstromkolonnen"

## Beschreibung

Die Erfindung betrifft ein Verfahren zur kontinuierlichen Veresterung von C₂-C₂₄-Fettsäuren mit C₁-C₅-Monoalkanolen oder C₂-C₃-Dialkanolen in flüssiger Phase bei erhöhten Temperaturen in Gegenwart von Katalysatoren, wobei man die Katalysatoren und die erwärmte Fettsäure auf den obersten Boden und die Alkanole dampfförmig oberhalb des Kolonnensumpfes an den untersten Boden einer mit einer Mehrzahl von Böden ausgestatteten Reaktionskolonne mit nachgeschalteten Rektifikationsteil gibt sowie die Fettsäuren und Alkanole im Gegenstrom bei einer Verweilzeit der Alkanole in der Flüssigphase der Reaktionskolonne von mindestens 20 Minuten umsetz, über den Rektifikationsteil ein Wasser/Alkanol-Gemisch entfernt und aus dem Kolonnensumpf den Fettsäureester abzieht.

Verfahren der vorstehend genannten Art zur kontinuierlichen Veresterung von Fettsäuren sind bekannt, vgl. H. Stage, Chemiker-Ztg./Chem.Apparatur 87, Nr. 18, 661 - 666 (1963). Die Veröffentlichung beschreibt die Veresterung von Fettsäuren mit Methanol und n-Butanol in bei Normaldruck betriebenen mehrteiligen Reaktionskolonnen mit Dampfprallböden. Üblicherweise erfolgt die technische kontinuierliche Fettsäureveresterung jedoch mehrstufig in Bodenkolonnen bei Drücken von 5 - 30 bar, vgl. DE-C 2503195.

Es wurde nun gefunden, daß es bei den bisher üblichen Verfahren zur kontinuierlichen Fettsäureveresterung zu unvertretbar starken Dehydratisierungserscheinungen kommt, z.B. beim Einsatz von verzweigten Monoalkanolen wie Isopropanol. Die Erfindung ist daher auf ein Verfahren gerichtet, mit dem diese unerwünschten Nebenreaktionen unterdrückt bzw. erheblich erniedrigt werden können.

Diese Aufgabe wird erfindungsgemäß durch ein Verfahren der eingangs genannten Art gelöst, das dadurch gekennzeichnet ist, daß man die Veresterung bei einem Kopfdruck der Reaktionskolonne von 200 - 900, vorzugsweise 700 - 900 hPa durchführt. Auf diese Weise läßt sich z.B. die Propenbildung, die gemäß dem Stand der Technik, bezogen auf eingesetztes Isopropanol, über 5 % beträgt, auf weniger als 0,1 % reduzieren. Zudem kann die Veresterung gemäß der Erfindung, im Gegensatz zu derjenigen gemäß DE-C-2503195, in einer einzigen Reaktionskolonne durchgeführt werden, wodurch die Investitionskosten erniedrigt werden. Mit dem Verfahren der Erfindung sind Umsetzungsgrade von mehr als 99 % erreichbar.

Als für das Verfahren der Erfindung geeignete Reaktionskolonnen sind allgemein sämtliche üblichen Bodenkolonnen wie Siebbodenkolonnen zu nennen, insbesondere jedoch Glockenbodenkolonnen mit hohen Flüssigkeitsständen. Typische Vertreter derartiger Kolonnen sind in der EP-B-0033929 sowie DE-A-3146142 beschrieben.

Gemäß einer bevorzugten Ausführungsform verwendet man das Verfahren der Erfindung zur Veresterung von C₆-C₂₀-Fettsäuren bzw. Gemischen derartiger Fettsäuren natürlichen, z.B. pflanzlichen, tierischen und/oder seetierischen oder synthetischen Ursprungs. Hierzu gehören insbesondere die bei der Aufarbeitung von Fettsäuregemischen natürlichen Ursprungs in großen Mengen anfallenden, sogenannten Vorlauffettsäuren mit 6 - 12 Kohlenstoffatomen.

Gemäß einer weiteren vorteilhaften Ausführungsform der Erfindung führt man die Veresterung mit C₂-C₅-Alkanolen, insbesondere Ethanol, Propanol, Butanol und Pentanol und Isomeren derselben durch, wobei man jedoch auch Methanol einsetzen kann, weiterhin mit Ethylenglykol oder Propylenglykol, wobei man Vollester und/oder Partialester dieser Dialkanole herstellen kann.

Gemäß einer weiteren vorteilhaften Ausführungsform der Erfindung führt man die Veresterung bei einer Temperatur der Reaktionskolonne von höchstens 190 ° C, insbesondere von 120-145 °C, durch. Diese im Vergleich zu üblichen Verfahren niedrigen Veresterungstemperaturen führen insbesondere zu einer Verringerung der für das Verfahren erforderlichen Energiekosten.

Gemäß einer weiteren vorteilhaften Ausführungsform der Erfindung verwendet man Veresterungskatalysatoren aus der von Schwefelsäure, Toluolsulfonsäure, Chlorsulfonsäure und Methylsulfonsäure gebildeten Gruppe. Besonders vorteilhaft ist p-Toluolsulfonsäure, die sich im Verfahrensverlauf in der Sumpfphase ansammelt und von dort in leichter Weise entfernt werden kann.

Gemäß einer weiteren vorteilhaften Ausführungsform der Erfindung setzt man Fettsäuren und Monoalkanole in molaren Verhältnissen von weniger als 1:2, insbesondere von 1:1,1 bis 1:1,8 und besonders bevorzugt von 1:1,2 bis 1:1,4 ein. Bereits mit diesen niedrigen molaren Verhältnissen erreicht man eine nahezu quantitative Umsetzung der eingesetzten Fettsäuren. Beim Einsatz von Dialkanolen werden die eingesetzten Molverhältnisse von Fettsäuren zu Dialkanolen, auch unter Berücksichtigung der gewünschten Produkte (Partial-und/oder Vollester) entsprechend angepaßt.

Gemäß einer weiteren vorteilhaften Ausführungsform der Erfindung gibt man die Fettsäuren in erhitzter Form in die Reaktionskolonne.

Gemäß einer weiteren vorteilhaften Ausführungsform der Erfindung gibt man das am Rektifikationsteil der Reaktionskolonne abgezogene Wasser/Alkanol-Gemisch, gegebenenfalls nach Abtrennung von überschüssigem Wasser, teilweise als Rückflußstrom an den Rektifikationsteil zurück; damit wird das Mitschleppen von Fettsäureester in dem Kopfprodukt verhindert.

Gemäß einer weiteren vorteilhaften Ausführungsform der Erfindung gibt man das am Rektifikationsteil abgezogene Wasser/Alkanol-Gemisch, gegebenenfalls nach Abtrennung von überschüssigem Wasser, mindestens teilweise als Azeotrop an einer Stelle in die Reaktionskolonne zurück, an der das molare Wasser/Alkanol-Verhältnis in der Kolonne ungefähr die Zusammensetzung des Azeotrops aufweist. Damit vermeidet man umständliche und kostenintensive Maßnahmen zur Entfernung des in den Azeotropen enthaltenen Wassers.

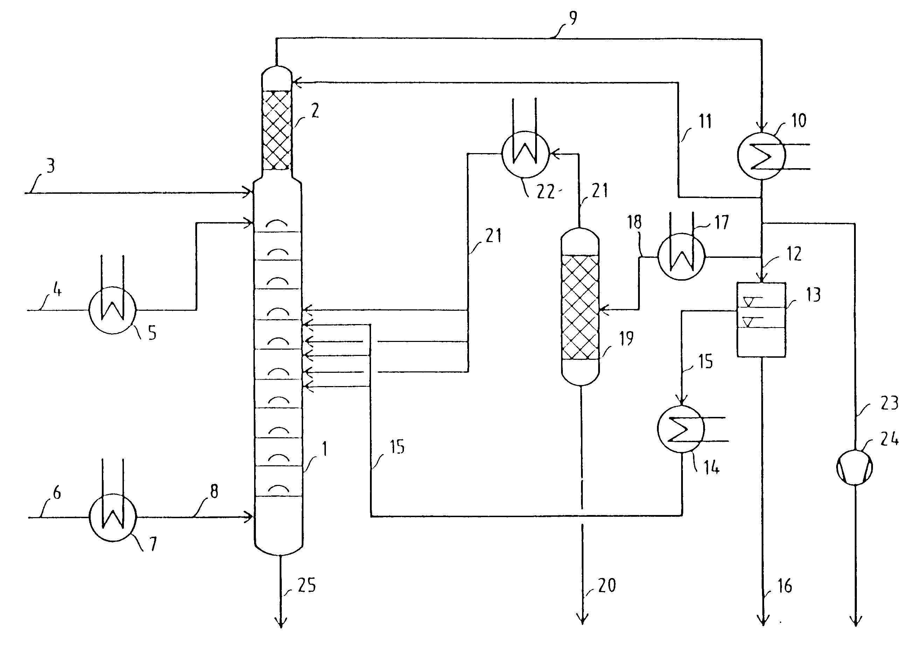

Das Verfahren der Erfindung wird im folgenden anhand einer bevorzugten Anlage und von Ausführungsbeispielen näher erläutert, wobei auf die Zeichnung Bezug genommen wird.

Die Zeichnung zeigt ein Schema zur kontinuierlichen Veresterung von Fettsäuren gemäß dem Verfahren der Erfindung.

Zentrales Element einer solchen Anlage ist eine eine Vielzahl von Glockenböden aufweisende Reaktionskolonne 1 mit aufgesetztem Rektifikationsteil 2. Katalysator bzw. Katalysatorlösung wird, gegebenenfalls nach Erhitzen, über eine Leitung 3 auf den obersten Boden der Reaktionskolonne 1 gegeben, ebenso wie die Fettsäure über eine Leitung 4, wobei die Fettsäure mittels eines Wärmeaustauschers 5 vor der Aufgabe auf die Reaktionskolonne erhitzt wird. Das eingesetzte Alkanol wird über eine Leitung 6 einem zweiten Wärmeaustauscher 7 zugeführt sowie überhitzt und über eine Leitung 8 unmittelbar oberhalb des Sumpfes der Reaktionskolonne 1 in dieselbe eingegeben.

Im Betrieb der Kolonne wird gebildetes Wasser/Alkanol-Gemisch am Kopf des Rektifikationsteiles 2 über eine Leitung 9 abgezogen und im Wärmetauscher 10 kondesiert. Ein Teil des Stromes wird über eine Leitung 11 dem Kopf des Rektifikationsteiles 2 wieder zugegeben, um das Mitschleppen von Fettsäuren über die Leitung 9 zu vermeiden.

Der andere Teil des Stroms der Leitung 9 wird bei Systemen mit mischungslücke über eine Leitung 12 einem Abscheider 13 zugeführt. Die leichtere organische Phase wird aus dem Abscheider über eine Leitung 15 nach Überhitzen in einem Wärmetauscher 14 an derjenigen Stelle in die Reaktionskolonne 1 eingespeist, an der das molare Wasser/Alkanol-Verhältnis in der Reaktionskolonne in etwa die Zusammensetzung des Stromes in der Leitung 15 aufweist. Die wäßrige Phase aus dem Abscheider 13 wird dem System über eine Leitung 16 entzogen.

Bei Systemen ohne Mischungslücke wird der Reststrom in einem Wärmetauscher 17 erhitzt und über eine Leitung 18 einer Rektifikationskolonne 19 zugeführt. Das wäßrige Sumpfprodukt wird dem System über eine Leitung 20 entzogen. Das Kopfprodukt, welches außer bei Methanol als Azeotrop anfällt, wird über eine Leitung 21 einem Wärmetauscher 22 zugeführt, in dem es überhitzt und dann analog dem Strom der Leitung 15 an derjenigen Stelle in die Reaktionskolonne 1 eingespeist wird, an der das molare Wasser/Alkanol-Verhältnis in der Reaktionskolonne in etwa die Zusammensetzung des Stromes der Leitung 21 aufweist.

Der Druck in der Keaktionskolonne 1 wird über eine Leitung 23 und eine Vakuumpumpe 24 eingestellt.

Das Sumpfprodukt der Reaktionskolonne 1, das im wesentlichen aus Fettsäureester, ggf. mit Katalysator besteht, wird über eine Leitung 25 abgezogen.

Bei stationären Betrieb der Reaktionskolonne findet auf den Böden die Veresterungsreaktion nach dem Gegenstromprinzip mit überlagerter Absorption/Desorption statt.

Die im folgenden beschriebenen Ausführungsbeispiele wurden in einer Kolonne ähnlich der in der DE-A-3146142 beschriebenen durchgeführt. Die Kolonne wies einen Durchmesser von 36 cm sowie 30 Böden mit 10 Glocken auf. Der aufgesetzte Rektifikationsteil umfaßte zwei Gewebepackungen (à 1,1 m) der Firma Montz.

### Beispiel 1:

Veresterung einer C₁₄-Fettsäure mit Rein-Isopropanol.

### Verfahrensparameter:

Molares Verhältnis Fettsäure/Rein-Isopropanol 1:1,8
Einsatztemperatur Rein-Isopropanol 140 °C
Einsatztemperatur der C₁₄-Fettsäure 135 °C
Verweilzeit der Fettsäure 3,3 h
Kolonnentemperatur 130 - 140 °C
Eingesetzter Katalysator: p-Toluolsulfonsäure, 1 Gew. % bezogen auf C₁₄-Fettsäure
Kopfdruck der Reaktionskolonne 800 hPa
Propenbildung, bezogen auf Isopropanol, weniger als 0,1 %
Bei Durchführung der Reaktion in derselben Reaktionskolonne bei leichtem Überdruck und entsprechend erhöhter Kolonnentemperatur stieg die Propenbildung, bezogen auf Isopropanol, auf über 5 %.

### Beispiel 2

Veresterung einer technischen C₁₄-Fettsäure mit Rein-Isopropanol und Isopropanol-Azeotrop

### Verfahrensparameter:

Molares Verhältnis C₁₄-Fettsäure: Rein-Isopropanol 1:1
Molares Verhältnis von C₁₄-Fettsäure: Isopropanol (Azeotrop) 1:0,5
Einsatztemperatur des Rein-Isopropanols 140 °C
Einsatztemperatur des Isopropanol-Azeotrops 120 °C
Einsatztemperatur des C₁₄-Fettsäure 135 °C
Verweilzeit der C₁₄-Fettsäure 3,3 h
Kolonnentemperatur 130 - 140 °C
Katalysator: p-Toluolsulfonsäure, 1 Gew. %, bezogen auf Fettsäure
Kopfdruck der Reaktionskolonne 800 hPa
Propenbildung: weniger als 0,1 % bezogen auf Isopropanol

## Patentansprüche

1. Verfahren zur kontinuierlichen Veresterung von C₂-C₂₄-Fettsäuren mit C₁-C₅-Monoalkanolen oder C₂-C₃-Dialkanolen in flüssiger Phase bei erhöhten Temperaturen in Gegenwart von Katalysatoren, wobei man die Katalysatoren und die erwärmte Fettsäure auf den obersten Boden und die Alkanole dampfförmig oberhalb des Kolonnensumpfes an den untersten Boden einer mit einer Mehrzahl von Böden ausgestatteten Reaktionskolonne mit nachgeschalteten Rektifikationsteil gibt sowie die Fettsäuren und Alkanole im Gegenstrom bei einer Verweilzeit der Alkanole in der Flüssigphase der Reaktionskolonne von mindestens 20 Minuten umsetzt, über den Rektifikationsteil ein Wasser/Alkanol-Gemisch entfernt und aus dem Kolonnensumpf den Fettsäureester abzieht, dadurch gekennzeichnet, daß man die Veresterung bei einem Kopfdruck der Reaktionskolonne von 200 - 900 hPa durchführt.

2. Verfahren nach Anspruch 1, dadurch gekennzeichnet, daß man die Veresterung bei einem Kopfdruck der Reaktionskolonne von 700 - 900 hPa durchführt.

3. Verfahren nach Anspruch 1 oder 2, dadurch gekennzeichnet, daß man C₆-C₂₀-Fettsäuren verestert.

4. Verfahren nach mindestens einem der Ansprüche 1 - 3, dadurch gekennzeichnet, daß man die Fettsäuren mit C₂-C₅-Alkanolen verestert.

5. Verfahren nach mindestens einem der Ansprüche 1 - 4, dadurch gekennzeichnet, daß man die Veresterung bei einer Temperatur der Reaktionskolonne von höchstens 190 °C, insbesondere von 120 - 145 °C durchführt.

6. Verfahren nach mindestens einem der Ansprüche 1 - 5, dadurch gekennzeichnet, daß man Veresterungskatalysatoren aus der von Schwefelsäure, Toluolsulfonsäure, Chlorsulfonsäure und Methylsulfonsäure gebildeten Gruppe verwendet.

7. Verfahren nach mindestens einem der Ansprüche 1 - 6, dadurch gekennzeichnet, daß man Fettsäuren und Monoalkanole in molaren Verhältnissen von weniger als 1:2, insbesondere von 1:1,1 bis 1:1,8, einsetzt.

8. Verfahren nach mindestens einem der Ansprüche 1 - 7, dadurch gekennzeichnet, daß man Fettsäuren und Monoalkanole in molaren Verhältnissen von 1:1,2 bis 1:1,4 einsetzt.

9. Verfahren nach mindestens einem der Ansprüche 1 - 8, dadurch gekennzeichnet, daß man das am Rektifikationsteil abgezogene Wasser/Alkanol-Gemisch, gegebenenfalls nach Abtrennung von überschüssigem Wasser, teilweise an den Rektifikationsteil zurückgibt.

10. Verfahren nach mindestens einem der Ansprüche 1 - 9, dadurch gekennzeichnet, daß man das am Rektifikationsteil abgezogene Wasser/Alkanol-Gemisch, gegebenenfalls nach Abtrennung von überschüssigem Wasser, mindestens teilweise als Azeotrop an einer Stelle in die Reaktionskolonne zurückgibt, an der das molare Wasser-/Alkanol-Verhältnis in der Kolonne annähernd die Zusammensetzung des Azeotrops aufweist.

## Claims

1. A process for the continuous esterification of C₂-C₂₄ fatty acids with C₁-C₅ monoalkanols or C₂-C₃ dialkanols in liquid phase at elevated temperature in the presence of catalysts, the catalysts and the heated fatty acid being fed in at the uppermost plate and the alkanols being introduced in the vapour phase above the sump at the lowermost plate of a multiple-plate reaction column followed by a rectifying section and the fatty acids and alkanols being reacted in countercurrent over a residence time of the alkanols in the liquid phase of the reaction column of at least 20 minutes, a water/alkanol mixture being removed through the rectifying section and the fatty acid ester being removed from the sump of the column, characterized in that the esterification is carried out at a head pressure of the reaction column in the range from 200 to 900 hPa.

2. A process as claimed in claim 1, characterized in that the esterification is carried out at a head pressure of the reaction column in the range from 700 to 900 hPa.

3. A process as claimed in claim 1 or 2, characterized in that C₆-C₂₀ fatty acids are esterified.

4. A process as claimed in at least one of claims 1 to 3, characterized in that the fatty acids are esterified with C₂-C₅ alkanols.

5. A process as claimed in at least one of claims 1 to 4, characterized in that the esterification is carried out at a temperature of the reaction column of at most 190°C and more especially at a temperature of 120 to 145°C.

6. A process as claimed in at least one of claims 1 to 5, characterized in that esterification catalysts from the group consisting of sulfuric acid, toluenesulfonic acid, chlorosulfonic acid and methylsulfonic acid are used.

7. A process as claimed in at least one of claims 1 to 6, characterized in that fatty acids and monoalkanols are used in molar ratios of less than 1:2 and more especially of 1:1.1 to 1:1.8.

8. A process as claimed in at least one of claims 1 to 7, characterized in that fatty acids and monoalkanols are used in molar ratios of from 1:1.2 to 1:1.4.

9. A process as claimed in at least one of claims 1 to 8, characterized in that the water/alkanol mixture removed from the rectifying section is partly returned to the rectifying section, optionally after separation of excess water.

10. A process as claimed in at least one of claims 1 to 9, characterized in that the water/alkanol mixture removed from the rectifying section, optionally after separation of excess water, is returned at least partly as an azeotrope to the reaction column at a point where the molar ratio of water to alkanol in the column has substantially the composition of the azeotrope.

## Revendications

1. Procédé d'estérification en continu d'acides gras en C₂ à C₂₄ avec des alcanols en C₁ à C₅ ou des dialcanols en C₂ - C₃ en phase liquide, à température élevée en présence de catalyseurs, procédé dans lequel on verse les catalyseurs et l'acide gras chauffé sur le plateau supérieur et les alcanols sous forme de vapeur au-dessus du pot de la colonne sur le plateau le plus bas d'une colonne de réaction pourvue d'une multiplicité de plateaux avec une partie de la rectification post-connectée et on fait réagir les acides gras et les alcanols à contre-courant, pour un temps de séjour des alcanols dans la phase liquide de la colonne de réaction d'au moins 20 minutes, on élimine par l'intermédiaire de la partie de la rectification un mélange eau/alcanol et on soutire du pot de la colonne l'ester d'acide gras, caractérisé en ce que l'on effectue l'estérification à une pression de tête de la colonne de réaction de 200 à 900 hPa.

2. Procédé selon la revendication 1, caractérisé en ce que l'on effectue l'estérification à une pression de tête de la colonne de réaction de 700 à 900 hPa.

3. Procédé selon la revendication 1 ou 2,caractérisé en ce que l'on estérifie des acides gras en C₆ à C₂₀.

4. Procédé selon au moins une des revendications 1 à 3, caractérisé en ce que l'on estérifie les acides gras avec des alcanols en C₂ à C₅.

5. Procédé selon au moins une des revendications 1 à 4, caractérisé en ce que l'on effectue l'estérification à une température de la colonne de la réaction d'au plus 190° C, en particulier de 120 à 145° C.

6. Procédé selon au moins une des revendications 1 à 5, caractérisé en ce que l'on utilise des catalyseurs d'estérification choisis dans le groupe formé par l'acide sulfurique, l'acide toluène sulfonique, l'acide chloro sulfonique et l'acide méthyle sulfonique.

7. Procédé selon au moins une des revendications 1 à 6, caractérisé en ce que l'on met en oeuvre des acides gras et des mono alcanols dans des rapports molaires inférieurs à 1:2, en particulier allant de 1:1,1 à 1:1,8.

8. Procédé selon au moins une des revendications 1 à 7, caractérisé en ce que l'on met en oeuvre des acides gras et des mono alcanols dans des rapports molaires allant de 1:1,2 à 1:1,4.

9. Procédé selon au moins une des revendications 1 à 8, caractérisé en ce que l'on reverse le mélange eau/alcanol soutiré à la partie de la rectification le cas échéant après séparation de l'eau excédentaire, partiellement sur la partie de rectification.

10. Procédé selon au moins une des revendications 1 à 9, caractérisé en ce que l'on reverse le mélange eau/alcanol soutiré sur la partie de rectification le cas échéant après séparation de l'eau en excès au moins sous forme d'azéotrope à un endroit de la colonne de réaction où le rapport molaire eau/alcanol dans la colonne, possède approximativement la composition de l'azéotrope.
